# EUROPEAN PATENT SPECIFICATION

(11) **EP 1 276 532 B1**
(45) Date of publication and mention of the grant of the patent: **21.09.2016**
(21) Application number: 01925833.4
(22) Date of filing: 19.04.2001
(51) Int. Cl.: A61M 25/08

(54) **SYSTEM AND METHOD FOR INTRAVASCULAR CATHETER NAVIGATION**
SYSTEM UND METHODE ZUM STEUERN EINES INTRAVASKULÄREN KATHETERS
SYSTEME ET PROCEDE DE NAVIGATION POUR UN CATHETER INTRAVASCULAIRE

(30) Priority: 21.04.2000 US 198736 P
(43) Date of publication of application: 22.01.2003
(60) Divisional application: 16182767.0
(73) Proprietor: Covidien LP, Mansfield, MA 02048 (US)
(72) Inventor: GILBOA, Pinhas, 34409 Haifa (IL)
(74) Representative: Soames, Candida Jane
(86) International application number: PCT/IL2001/000363
(87) International publication number: WO 2001/080922

(56) References cited:
- WO-A-96/05768
- WO-A-97/44089
- US-A- 5 476 100
- US-A- 5 919 147

## Description

### FIELD AND BACKGROUNID OF THE INVENTION

The present invention relates to catheter systems and, in particular, it concerns systems for navigating catheters to a given location within the body.

A wide range of procedures performed by medical practitioners require navigation of a catheter through complex features of the vascular system to reach a specific location within the body. In certain cases, this navigation may be difficult and time consuming, adding to the cost and risks of the procedure.

By way of example, various medical procedures require wires to be introduced through the coronary sinus (CS) ostium (Os). An example of such procedures is installation of a bi-atrial or bi-ventricular implantable device, such as a dual sided pacing and/or defibrillating system, in which leads must be placed inside the CS in electrical contact with the left ventricle.

Dual sided pacing and/or defibrillating systems are described in the patent literature (e.g. US patent 5,902,324 to Thompson et al, WO 99/55415 to Struble et al. and WO 99/30777 to Bakels et al.) and are commercially available. Commercially available examples include the Contak™ CD congestive heart failure device (CHFD) using the EASYTRAK™ lead system available from Guidant, and the InSync Implantable Cardiac Defibrillator (ICD) using Attain™ Side-Wire (SD) lead system available from Medtronic. In both systems, guidance of the lead to the CS Os is aided by a special introducer sheath that has a predetermined curve which fits to the anatomy of the "average person". An example for such a sheath is VENAPORT™ manufactured by Cardima. However, in many cases the shape of the sheath, as created for the "average" anatomy, does not fit to the anatomy of the individual patient. If the end portion of the sheath is not easily guided into the CS Os, than it is necessary to manipulate it until it gets there by pushing and rotating it. Searching for the ostium using only X-ray imaging as guidance, as it is currently done, results in extending the duration of the procedure and exposing the patient to unnecessary X-ray radiation.

A wide range of steerable catheter devices are known. Probably the most relevant known to the applicant is U.S. Patent No. 5,636,634 to Kordis et al. in which a steerable first catheter directs the introduction of a guiding sheath, where such sheath can otherwise be free of any onboard steering mechanism. The guiding sheath subsequently directs the introduction of the electrode-carrying second catheter, which can likewise be free of any onboard steering mechanism. Kordis does not, however, provide for any sensing element to locate the distal end portion of the catheter, nor for registration of the location of the catheter and an image of the vessels.

U.S. Patent No. 5,599,305 to Hermann et al. describes a catheter-introducing system which includes a large-diameter introducer catheter comprising a flexible sheath and an obturator having a steering mechanism operated by a proximal actuator handle. The catheter introducer system is usually introduced with the obturator inside of the flexible sheath so that the obturator can effect steering by laterally deflecting the distal end of the combined sheath and obturator. This catheter-introducing system is particularly useful for large-diameter sheaths that are not readily introduced over guide wires.

U.S. Patent No. 5,386,828 to Owens at al. describes an apparatus that helps to detect the location of a guide wire within the body of a patient. The apparatus includes a guide wire with an internally housed sensing element. That sensing element may be detected by an external transponder, and a rough estimation of its location relative to the anatomy can be produced. Other than measuring the location of a portion of the guide wire inside the body, the guide wire is used as it would normally be used in any regular over-the-wire intravascular procedure, i.e., pushing the wire into the blood vessel until it reaches a target position and then sliding the desired component to be installed along the wire toward its end.

The Owens et al. apparatus has no embedded steering mechanism. In addition, the sensing element position is not integrated with an imaging device or other system for locating anatomical features such that the apparatus is not particularly helpful in navigating to a particular anatomical feature. Finally, the Owens apparatus does not provide position information in six degrees of freedom.

There is therefore a need for a catheter navigation system and method which would combine position measurement with advantageous steering features to facilitate navigation through convoluted features of the vascular system, such as the CS Os. It would also be highly advantageous to provide such a system and method in which at least some of the position measuring and/or steering components could be withdrawn from the catheter once m position to allow use of the full capacity of a small dimension lumen.

WO9605768 (A1) discloses a locating system for determining the location and orientation of an invasive medical instrument, for example a catheter or endoscope, relative to a reference frame, comprising: a plurality of field generators which generate known, distinguishable fields, preferably continuous AC magnetic fields, in response to drive signals; a plurality of sensors situated in the invasive medical instrument proximate the distal end thereof which generate sensor signals in response to said fields; and a signal processor which has an input for a plurality of signals corresponding to said drive signals and said sensor signals and which produces the three location coordinates and three orientation coordinates of a point on the invasive medical instrument.

### SUMMARY OF THE INVENTION

The present invention is a system for intravascular catheter navigation. According to the teachings of the present invention there is provided, a system for leading a sheath to a location inside a cavity in the body as defined in claim 1.

According to a further feature of the present invention, the at least one steering mechanism includes a steering mechanism incorporated into the sheath.

According to a further feature of the present invention, the at least one steering mechanism includes a distal steering mechanism incorporated into the center-support.

According to a further feature of the present invention, the distal steering mechanism has a length of not more than 1 cm.

According to a further feature of the present invention, the distal steering mechanism is located distally of the position sensor.

According to a further feature of the present invention, the distal steering mechanism includes a soft elastic material which is free to bend when not actuated, and is bent when actuated.

According to a further feature of the present invention, the at least one steering mechanism further includes a proximal steering mechanism incorporated into the center-support proximally to the position sensor.

According to a further feature of the present invention, a minimum radius of curvature produced in a first region of the center-support by full actuation of the proximal steering mechanism is at least five times greater than a minimum radius of curvature produced in a second region of the center-support by full actuation of the distal steering mechanism.

According to a further feature of the present invention, the at least one steering mechanism further includes a second steering mechanism incorporated into the sheath.

According to a further feature of the present invention, the at least one steering mechanism includes a soft elastic material which is free to bend when not actuated, and is bent when actuated.

According to a further feature, the present invention is adapted for measuring the position of the sensor in at least a rotation angle of the sensor along the length of the center-support.

According to a further feature, the present invention is adapted for measuring the position of the sensor in at least three degrees of freedom.

According to a further feature, the present invention is adapted for measuring the position of the sensor in six degrees of freedom.

According to a further feature of the present invention, navigation of the position sensor is performed by bending the distal portion of the center-support towards a branched vessel and advancing the sheath over the center-support into the vessel.

According to a further feature of the present invention, registration of the image to the system of coordinates is performed by incorporating a position sensor to the imaging device, the position sensor measuring the six degrees of freedom location of the imaging device in the same reference system of coordinates.

According to the teachings of the present invention, the sheath is may be (a) a steerable catheter having a lumen, a steerable distal portion, a proximal portion configured to be held by the practitioner and a first steering mechanism manually operable from the proximal portion so as to steer the distal portion of the catheter; and (b) the center-support is a steerable center-support deployed within, but retractable from, the lumen, a steerable guidewire having a steerable distal portion, a proximal portion configured to be accessible to the practitioner and a second steering mechanism manually operable from the proximal portion so as to steer the distal portion of the center-support.

According to a further feature of the present invention, the center-support further includes the position sensor mounted within the distal portion, the position sensor forming part of a location determining system for determining a position of at least one point on the distal portion relative to the frame of reference.

According to a further feature of the present invention, a minimum radius of curvature produced in a first region of the catheter system by full actuation of the first steering mechanism is at least five times greater than a minimum radius of curvature produced in a second region of the catheter system by full actuation of the second steering mechanism.

According to a further feature of the present invention, the position sensor is part of a six-degrees-of-freedom position sensing system.

### BRIEF DESCRIPTION OF THE DRAWINGS

The invention is herein described, by way of example only, with reference to the accompanying drawings, wherein:
FIG. 1 is a schematic representation of a catheter system, constructed and operative according to the teachings of the present invention, for determining the coordinates of a feature within the body;
FIGS. 2A and 2B are schematic representations showing two stages of navigation of a catheter, constructed and operative according to the teachings of the present invention, to the coronary sinus ostium;
FIG. 3 is a schematic partially-cut-away view of a distal portion of the catheter of Figures 2A and 2B including a position sensor within a lumen;
FIG. 4 is a schematic cross-sectional view illustrating a first mechanism for locking the position sensor in a given position with the lumen of Figure 3;
FIG. 5 is a schematic cross-sectional view illustrating a second mechanism for locking the position sensor in a given position with the lumen of Figure 3;
FIG. 6 is a schematic longitudinal cross-sectional view illustrating a preferred implementation of a catheter constructed and operative according to the teachings of the present invention including a two-stage steering mechanism;
FIG. 7 is a schematic longitudinal cross-sectional view illustrating an alternative preferred implementation of a catheter constructed and operative according to the teachings of the present invention including a two-stage steering mechanism; and
FIGS. 8A-8C illustrate a sequence of stages in a preferred mode of navigation using a distal steering mechanism of the two-stage steering mechanism of Figures 6 or 7.

### DESCRIPTION OF THE PREFERRED EMBODIMENTS

The present invention is a system for intravascular catheter navigation.

The principles and operation of systems according to the present invention may be better understood with reference to the drawings and the accompanying description.

Although the present invention may be used to advantage in a wide range of procedures, it will be described by way of non-limiting example in the context of procedures in which a catheter is to be inserted through the coronary sinus. Thus, a first aspect of the invention will be illustrated in the context of navigating a wire into the CS Os (coronary sinus ostium), while a second aspect of the invention will be illustrated in the context of navigating a wire within the CS (coronary sinus) tree.

One aspect of the present invention provides an apparatus that employ a positioning sensor, preferably a six degrees-of-freedom positioning sensor, temporarily associated with an intra-body delivery mechanism or "sheath", to assist during navigation of the delivery mechanism. This avoids the need to incorporate the sensor into the delivery mechanism itself.

Thus, in one preferred implementation, a method for leading a sheath to a location inside a cavity in the body is disclosed, by: using an imaging device for acquiring an image of a portion of the body, the image being registered to a reference system of coordinates; acquiring coordinates of at least one location within the portion of the body; securing a position sensor at a distal end portion of a sheath, the position sensor measuring a position of the end portion of the sheath in the reference frame of coordinates; and navigating the position sensor to the coordinates of at least one location within the portion of the body.

In a preferred implementation, the method also includes retracting the position sensor from the sheath to free the lumen for inserting an apparatus into the lumen, thereby facilitating leading of the apparatus to the location at the portion of the body.

In structural terms, the invention provides a device for leading an apparatus to a target position inside the body, the device having: a sheath having a lumen; a center-support incorporating a position sensor at its distal end portion, the center-support being insertable from a proximal portion of the sheath into the lumen and being retractable from the lumen through the proximal portion of the sheath; and at least one steering mechanism for co-deflecting the sheath and the center-support.

Preferably, the position sensor is configured to provide an indication of the rotational position of the sensor around a central axis of the center-support, thereby facilitating operation of the steering mechanism. Additionally, or alternatively, the position sensor is configured to provide an indication of the position of the sensor in at least three degrees of freedom. Most preferably, the sensor provides position information in six degrees of freedom. A preferred example of a sensor, and the accompanying elements of a system, for measuring the six degrees-of-freedom coordinates of a miniature positioning sensor incorporated into an intra-body probe is described in PCT publication no. WO00/10456. Accordingly, details of the position sensor system will not be described here in detail. PCT publication no. WO00/16684 teaches methods and apparatus to navigate a probe inside the body. In particular, by using an echo-imaging device that is equipped with a positioning sensor, the coordinates of a feature of interest, such as the CS Os, can be acquired. By equipping a probe with a positioning sensor, the apparatus may also assist in navigation of the probe to the feature of interest.

Optionally, registration of the image to the reference system of coordinates may be performed by incorporating an additional position sensor into the imaging device. In this case, the additional position sensor preferably measures the six degrees of freedom location of the imaging device in the same reference system of coordinates.

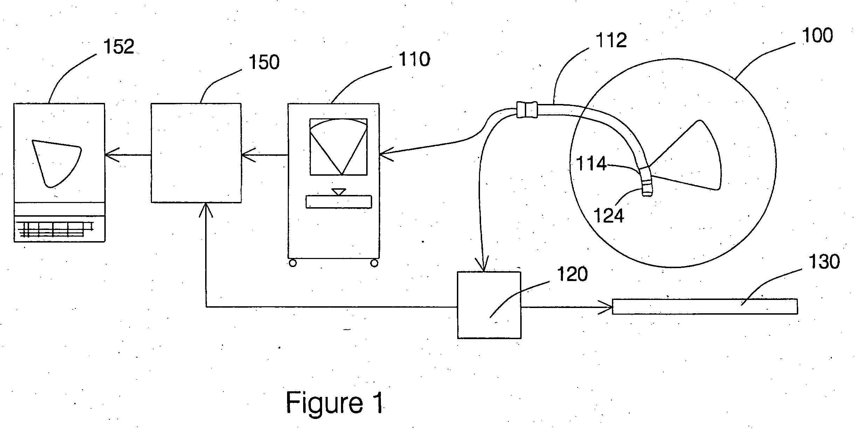

Turning now to Figure 1, this shows a system for determining the coordinates of the CS Os according to this invention. An intra-cardiac echo catheter **112,** such as ICE™ manufactured by Boston Scientific, having an image transducer **114** is modified by the addition of a six-degrees-of-freedom positioning sensor **124.** A positioning system **120** feeds currents to transmitting antennae **130,** with such antennae transmitting electromagnetic fields into the body of the patient **100.** The signals sensed by sensor **124** are sent to the positioning system, which determines the six degrees-of-freedom (i.e., location in x, y, z and orientation in roll, pitch, yaw) of the sensor. The image generated by the image generator unit **110,** together with the coordinates of the imaging probe, are fed into the system's control and computation unit **150.** Unit **150** can be any computer, for example a personal computer. The image is displayed on the computer monitor **152.** Using a standard pointing device such as a mouse or touch screen, it is possible to acquire the coordinates of a landmark, in this example, the coronary sinus ostium position. Other types of echo imaging devices can also be used in the same manner. More-fully detailed information can be found in the aforementioned PCT publications nos. WO00/10456 and WO00/16684, as well as in PCT publication no. WO01/12057.

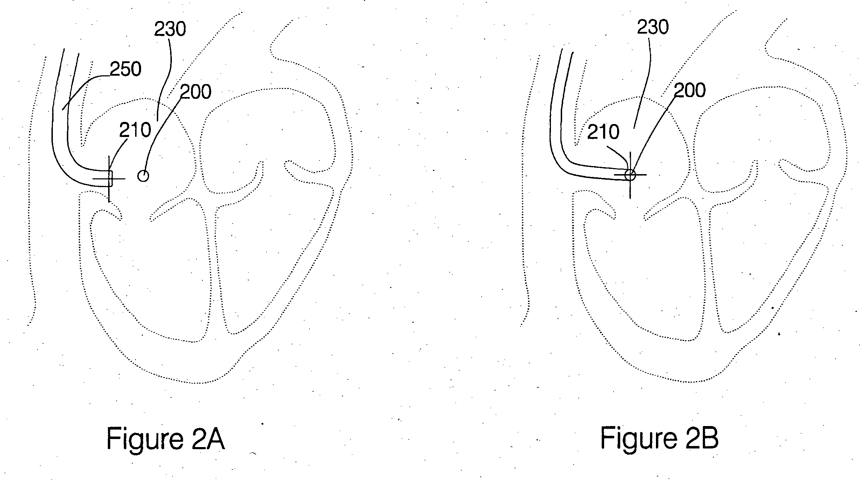

In order to direct a sheath into the CS Os, a positioning sensor is temporarily associated with a distal portion of the sheath. At least one steering mechanism is also provided. The resulting steerable-trackable sheath **250** is inserted into the right atrium **230** as shown in Figure 2a. The distal portion **210** of the sheath, together with the ostium **200,** is displayed on the computer screen **152,** preferably in two perpendicular projections. Navigating the sheath to the ostium is easily performed by steering the sheath while directing symbols **200** and **210** on the computer screen to coincide, as can be seen in Figure 2b.

It should be noted that the at least one steering mechanism may be incorporated either into the sheath or a separate element deployed therewithin. Where two steering mechanisms are provided, a further possibility of dividing the steering mechanisms between the sheath and a separate element also falls within the scope of the present invention.

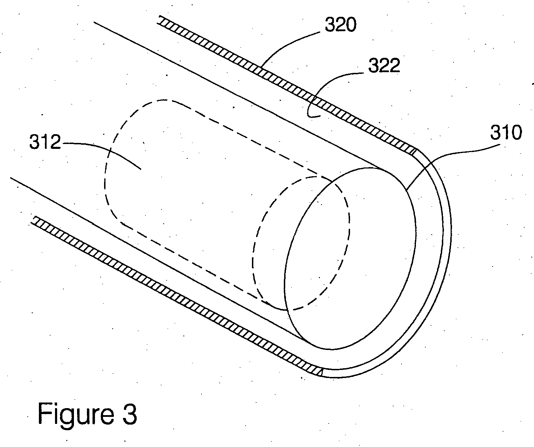

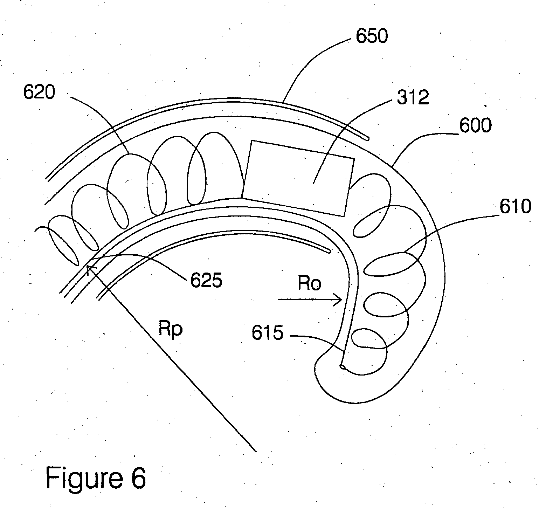

By way of a first non-limiting example, Figures 3-5 illustrate a basic implementation in which a steering mechanism is incorporated into the sheath. A preferred set of implementations in which at least one steering mechanism is implemented as part of a separate element will then be described with reference to Figures 6-8.

Turning now to the implementation of Figure 3, the sheath of this embodiment may optionally be implemented using a commercially available steerable sheath such as, for example, a sheath available from Cardima under the name NAVIPORT™. A positioning sensor **312** is mounted at the distal portion of a flexible tube **310.** Prior to the use of the sheath, the tube is inserted into the lumen **322** of sheath **320,** and it's distal end portion securely hold in place.

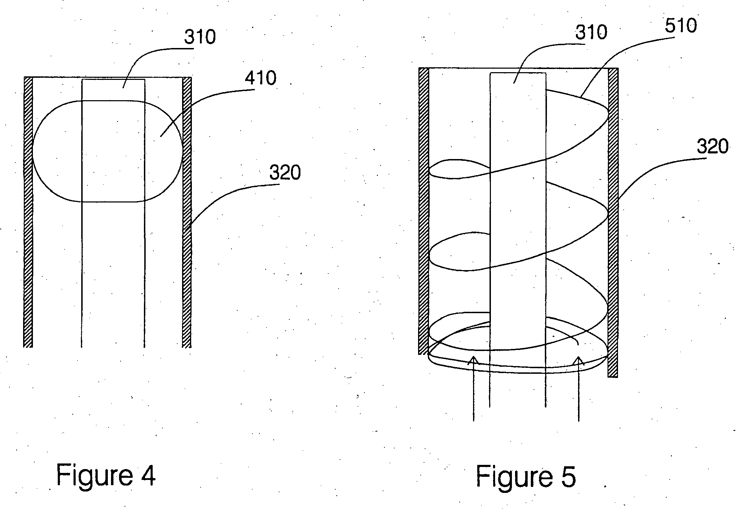

During navigation of the device into position, position sensor **312** is preferably held in a constant position relative to sheath **320,** typically by use of a locking mechanism incorporated within the distal portion of tube **310.** Various known mechanisms are suited for this task. By way of example, Figure 4 shows an inflatable balloon **410** deployed around tube **310.** When inflated, balloon **410** is pushed against sheath **320,** holding the tube in a fixed position and orientation relative to the sheath. In an alternative example of a locking mechanism, Figure 5 shows a coil **510** that is attached to the distal end of tube **310.** When coil **510** is compressed, its diameter increases. Hence the tube is securely held in place by compressing the coil and is released by releasing the compression force.

After the sheath is navigated to the required feature, such as the CS Os, the locking mechanism is released and tube **310** is retracted, thereby freeing the entire inner lumen of the sheath for use in a subsequent step of the procedure, for example, for delivering an electrical lead to the CS.

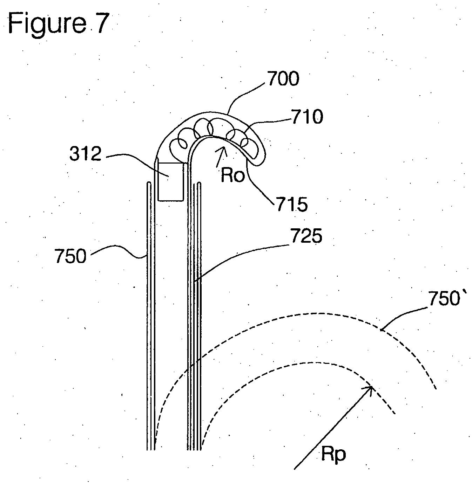

Turning now to Figures 6-8, there are shown two examples from a preferred set of implementations in which at least one steering mechanism is implemented as part of a separate element, referred to as a center-support. In the example of Figure 6, the center-support features two independently operable steering mechanisms while the sheath is passive in the steering process. In the example of Figure 7, the center-support features a singe steering mechanism, preferably supplemented by a steering mechanism in the sheath, as will be described.

In both cases, the central-support here preferably includes a distal steering mechanism which is small (preferably with a length of not more than 1 cm), preferably located distally of the position sensor. This distal steering mechanism preferably includes a soft elastic material which is free to bend when not actuated, and is bent when actuated, rendering it highly suited to navigation along convoluted pathways such as the coronary sinus tree.

Turning now specifically to Figure 6, this shows a preferred implementation of the catheter system with a center-support including a proximal steering mechanism proximally to the position sensor. Thus, a center support **600,** having a small diameter preferably of less than 1.8 mm, is inserted into a thin sheath **650.** Dual steering mechanisms are incorporated into that center support. A position sensor **312** is incorporated into the distal portion of the center support between the two steering mechanisms. A distal steering mechanism is incorporated into the distal end portion of the center support, employing a short and very soft spring coil **610,** preferably in a diameter of 1 mm and a length of 5 mm, and an off-centered steering wire **615** attached to the end of the spring. The spring coil is made of elastic material, and in a shape that allows it to bend freely while the steering mechanism is not actuated. By pulling wire **615,** the end portion of center support bends, having a small radius, preferably of less than 3 mm. When the steering wire is released, the distal end portion of the center support is kept soft and bendable, thus suitable for use as a guide wire through the blood vessels. A second, proximal steering mechanism is incorporated into the other side of the position sensor **312** within the center-support. This includes a spring coil **620,** preferably of length about 60 mm. By pulling the off-centered steering wire **625,** coil **620** is bent, thereby causing the center-support and outer sheath to bend similarly.

It should be noted that the distal and proximal steering mechanisms are preferably of significantly different dimensions suited to performing different functions in the navigation process. Specifically, the proximal steering mechanism is typically a significantly longer and larger-radius steering mechanism suited for steering through primary blood vessels and body cavities while the distal steering mechanism is typically a short, small-radius steering mechanism particularly suited for navigating within small networks of vessels such as the coronary sinus tree. In numerical terms, it is typically preferred that the ratio between the radius of curvature R_{P} of the proximal steering mechanism and the radius of curvature R_{D} of the distal steering mechanism (each measured in its fully actuated state) is at least 5:1, and more preferably, at least 10:1.

Turning now to Figure 7, this shows an alternative implementation in which the proximal steering mechanism is incorporated into the sheath. Specifically, a flexible outer sheath **750** may be deflected to a position shown by dashed lines **750**' by tension applied to control wire **725.** As before, a central-support **700** has a distal steering mechanism made up of a soft spring **710** and control wire **715** deployed distally with respect to position sensor **312.** This configuration is generally analogous to that of Figure 6, but provides an added controllable parameter by allowing adjustment of the distance between, or of a degree of overlap between, the proximal and distal steering mechanisms.

Turning now to the operation of the embodiments of Figures 6 and 7, in each case the combined sheath and the center-support is navigated to the CS Os using the proximal steering mechanism to deflect the distal portion of the sheath toward the ostium. Unlike the earlier embodiment of Figures 3-5, the wall of the sheath is here preferably sufficiently thin to be pushed further into the CS. Before doing so, the center support may advantageously be replaced momentarily by a special catheter such as VEEPORT™ manufactured by Cardima, used to inject contrast agent to the CS, thus making it possible to achieve good contrast image of the CS tree. That image is registered to the positioning system such as by the methods described in PCT publications nos. WO00/16684 and WO01/12057. After producing an improved contrast image, the above-mentioned special catheter is pulled out of the sheath and the center support is replaced within the sheath with its distal end containing the distal steering mechanism extending ahead of the end of the sheath. The high flexibility of the center support's distal end allows the apparatus to be pushed into the CS without the fear of puncturing the vessel.

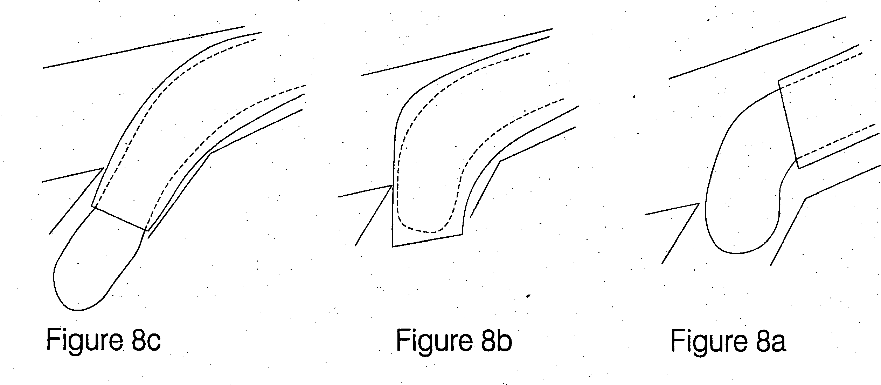

A preferred procedure for maneuvering the apparatus into a branch of the CS tree will now be described with reference to Figures 8A-8C. Firstly, the center support is rotated until the bending direction of the distal steering mechanism is in the direction of the branch vessel. This is done by measuring the direction of the branch opening, as known from the image data, relative to the steering mechanism as measured by positioning sensor **312.** Then, the distal steering mechanism is deflected into the opening (Figure 7A). The sheath is then pushed forward over the center support into the branched vessel (Figure 7B). The tension is then released from the control wire and the center support is pushed forward so that it once again extends ahead from the distal end of the sheath (Figure 7C).

Once the sheath is located in the desired branch of the CS, the center support can be retracted, and the wire to be implanted may easily be inserted in its place.

Many other intra-body medical treatments can be performed similarly. For instance, leading an introducer sheath trans-septaly from the right atrium into the left atrium. Under the current invention that is done by taking an image that is registered to the reference system of coordinates as defined by the positioning system, marking a coordinate on the septum, preferably the fossa ovalis, to be penetrate through, insert a tube having a position sensor inside the lumen of an introducer sheath, leading the tip of the sheath to the said coordinate on the septum, replacing the tube having the positioning sensor by a long needle and puncturing the septum by pushing the sheath trough it.

It will be appreciated that the above descriptions are intended only to serve as examples, and that many other embodiments are possible within the scope of the present invention.

## Claims

1. A system for leading a sheath to a location inside a cavity in the body, comprising:
(a) an imaging device (114) capable of acquiring an image of a portion of the body and acquiring the coordinates of at least one location within said portion of the body;
(b) a sheath (320; 650; 750) housing a center-support (310; 600; 700) with a position sensor (312) attached to a distal portion thereof and at least one steering mechanism (610, 615; 620, 625; 710, 715, 725) for co-deflecting said sheath (320; 650; 750) and said center-support (310; 600; 700); and
(c) a processor capable (150, 152) of displaying information from said position sensor (312) relating to the position and orientation of the position sensor (312),
wherein the position sensor (312) is located at a distal end portion of said sheath (320; 650; 750) and wherein said image and said displayed information are all registered to a common reference system of coordinates.

2. The system of claim 1 wherein said position sensor (312) is retractable from said sheath (320; 650; 750) thereby freeing a lumen within said sheath (320; 650; 750) for inserting an apparatus into said lumen, thereby facilitating leading of said apparatus through said sheath (320; 650; 750) to a distal end thereof.

3. The system of claim 1 wherein said sheath (320; 650; 750) has a lumen, said center-support (310; 600; 700) being insertable from a proximal portion of said sheath (320; 650; 750) into said lumen and retractable from said lumen through said proximal portion of said sheath (320; 650; 750), and
wherein said center-support (310; 600; 700) incorporates said position sensor (312) at its distal position.

4. The system of claim 3, wherein said at least one steering mechanism includes a steering mechanism incorporated into said sheath (320).

5. The system of claim 3, wherein said at least one steering mechanism (610, 615; 620, 625; 710, 715, 725) includes a distal steering mechanism (610, 615; 710, 715) incorporated into said center-support (600; 700).

6. The system according to either claim 1 or 3, adapted for measuring the position of the sensor (312) in at least a rotation angle of the sensor along the length of the center-support (310; 600; 700).

7. The system according to either claim 1 or 3, adapted for measuring the position of the sensor (312) in at least three degrees of freedom.

8. The system according to either claim 1 or 3, adapted for measuring the position of the sensor (312) in six degrees of freedom.

9. The system of claim 1, further comprising a second position sensor (124) associated with said imaging device (114), said second position sensor (124) measuring the six degrees of freedom location of said imaging device (114) in the same reference system of coordinates.

## Patentansprüche

1. Ein System um eine Hülle an eine Stelle innerhalb einer Körperhöhle zu bringen, bestehend aus:
(a) einem Bildgebungsgerät (114), fähig zur Aufnahme eines Bildes eines Körperabschnittes und Erfassung der Koordinaten mindestens einer Position innerhalb dieses Körperabschnittes;
(b) einer Hülle (320; 650; 750), in der eine Mittelstütze (310; 600; 700) untergebracht ist, mit einem Positionssensor (312), befestigt an seinem distalen Abschnitt und mindestens einem Steuerungsmechanismus (610, 615; 620, 625; 710, 715, 725) zur Co-Ablenkung dieser Hülle (320; 650; 750) und dieser Mittelstütze (310; 600; 700); und
(c) einem Prozessor, fähig (150, 152) zur Anzeige von Information von diesem Positionssensor (312), über die Position und Ausrichtung des Positionssensors (312),
wobei sich der Positionssensor (312) an einem distalen Ende dieser Hülle (320; 650; 750) befindet und wobei dieses Bild und die genannte angezeigte Information alle in einem gemeinsamen Koordinaten - Referenzsystem registriert werden.

2. Das System nach Anspruch 1, wobei der genannte Positionssensor (312) aus dieser Hülle (320; 650; 750) zurückgezogen werden kann, wodurch ein Lumen innerhalb dieser Hülle (320; 650; 750) frei wird, um eine Vorrichtung in dieses Lumen einzuführen, wodurch die Führung dieser Vorrichtung durch diese Hülle (320; 650; 750) an ihr distales Ende erleichtert wird.

3. Das System nach Anspruch 1, wobei die genannte Hülle (320; 650; 750) ein Lumen aufweist und die genannte Mittelstütze (310; 600; 700) von einem proximalen Teilstück dieser Hülle (320; 650; 750) aus, in dieses Lumen eingeführt werden kann und über dieses Lumen aus diesem proximalen Teilstück dieser Hülle (320; 650; 750) entfernt werden kann, und
wobei diese Mittelstütze (310; 600; 700) diesen Positionssensor (312) an seinem distalen Ende umfasst.

4. Das System nach Anspruch 3, wobei der genannte, mindestens einen Steuerungsmechanismus einen Steuerungsmechanismus umfasst, der in diese Hülle (320) integriert ist.

5. Das System nach Anspruch 3, wobei der genannte, mindestens eine Steuerungsmechanismus (610, 615; 620, 625; 710, 715, 725) einen distalen Steuerungsmechanismus (610, 615; 710, 715) umfasst, der in diese Mittelstütze (600; 700) integriert ist.

6. Das System nach Anspruch 1 oder 3, geeignet zur Messung der Position des Sensors (312) in mindestens einem Drehwinkel des Sensors entlang der Mittelstütze (310; 600; 700).

7. Das System nach Anspruch 1 oder 3, geeignet zur Messung der Position des Sensors (312) in mindestens drei Freiheitsgraden.

8. Das System nach Anspruch 1 oder 3, geeignet zur Messung der Position des Sensors (312) in sechs Freiheitsgraden.

9. Das System nach Anspruch 1, weiterhin umfassend einen zweiten Positionssensor (124), kombiniert mit dem genannten Bildgebungsgerät (114), dieser zweite Positionssensor (124) misst die sechs Freiheitsgrad- Position dieses Bildgebungsgerätes (114) im selben Koordinatenreferenzsystem.

## Revendications

1. Système pour mener une gaine jusqu'à un emplacement à l'intérieur d'une cavité dans le corps, comprenant :
(a) un dispositif d'imagerie (114) susceptible d'acquérir une image d'une partie du corps et d'acquérir les coordonnées d'au moins un emplacement à l'intérieur de ladite partie du corps ;
(b) une gaine (320 ; 650 ; 750) logeant un support central (310 ; 600 ; 700) avec un capteur de position (312) attaché à une partie distale de ce dernier et au moins un mécanisme d'orientation (610, 615 ; 620, 625 ; 710, 715, 725) pour faire dévier conjointement ladite gaine (320 ; 650 ; 750) et ledit support central (310 ; 600 ; 700) ; et
(c) un processeur (150, 152) susceptible d'afficher des informations provenant dudit capteur de position (312) se rapportant à la position et à l'orientation du capteur de position (312),
dans lequel le capteur de position (312) est situé au niveau d'une partie d'extrémité distale de ladite gaine (320 ; 650 ; 750) et dans lequel ladite image et lesdites informations affichées sont toutes enregistrées sur un système de coordonnées de référence commun.

2. Système selon la revendication 1, dans lequel ledit capteur de position (312) est rétractable depuis ladite gaine (320 ; 650 ; 750) libérant ainsi une lumière à l'intérieur de ladite gaine (320 ; 650 ; 750) pour insérer un appareil dans ladite lumière, facilitant ainsi l'avancement dudit appareil à travers ladite gaine (320 ; 650 ; 750) jusqu'à une extrémité distale de celle-ci.

3. Système selon revendication 1, dans laquelle ladite gaine (320 ; 650 ; 750) a une lumière, ledit support central (310 ; 600 ; 700) pouvant être inséré à partir d'une partie proximale de ladite gaine (320 ; 650 ; 750) dans ladite lumière et étant rétractable depuis ladite lumière à travers ladite partie proximale de ladite gaine (320 ; 650 ; 750) et
dans lequel ledit support central (310 ; 600 ; 700) incorpore ledit capteur de position (312) au niveau de sa position distale.

4. Système selon la revendication 3, dans lequel ledit au moins un mécanisme d'orientation inclut un mécanisme d'orientation incorporé dans ladite gaine (320).

5. Système selon la revendication 3, dans lequel ledit mécanisme d'orientation (610, 615 ; 620, 625 ; 710, 715, 725) inclut un mécanisme distal d'orientation (610, 615 ; 710, 715) incorporé dans ledit support central (600 ; 700).

6. Système selon l'une ou l'autre de la revendication 1 ou 3, conçu pour mesurer la position du capteur (312) dans au moins un angle de rotation du capteur le long de la longueur du support central (310 ; 600 ; 700).

7. Système selon l'une ou l'autre de la revendication 1 ou 3, conçu pour mesurer la position du capteur (312) dans au moins trois degrés de liberté.

8. Système selon l'une ou l'autre de la revendication 1 ou 3, conçu pour mesurer la position du capteur (312) dans six degrés de liberté.

9. Système selon la revendication 1, comprenant en outre un second capteur de position (124) associé audit dispositif d'imagerie (114), ledit second capteur de position (124) mesurant les six degrés de liberté au niveau de l'emplacement dudit dispositif d'imagerie (114) dans le même système de coordonnées de référence.
